# EUROPEAN PATENT APPLICATION

(11) **EP 4 458 279 A1**
(43) Date of publication of application: **06.11.2024**
(21) Application number: 22913658.5
(22) Date of filing: 13.10.2022
(51) Int. Cl.: A61B 17/115

(54) **DEFLECTION JOINT STRUCTURE, STAPLER AND MEDICAL EQUIPMENT**

(30) Priority: 31.12.2021 CN 202111683611
(71) Applicant: Surgnova Healthcare Technologies (Zhejiang) Co., Ltd., Ningbo, Zhejiang 315300 (CN)
(72) Inventor: LI, Guangrong, Ningbo Zhejiang 315300 (CN); LI, Yang, Ningbo Zhejiang 315300 (CN)
(74) Representative: Altmann Stößel Dick Patentanwälte PartG mbB
(86) International application number: PCT/CN2022/125149
(87) International publication number: WO 2023/124394

(57) **Abstract**

Provided are a deflection joint structure (100), a stapler, and a medical device are provided. The deflection joint structure (100), achieving a deflection of a deflection component (A) relative to a fixed component (B), including a first connecting portion (101), a second connecting portion (102), and a joint portion (103). A part of the joint portion (103) is provided in the first connecting portion (101), and another part of the joint portion (103) is provided in the second connecting portion (102). The joint portion (103) includes a plurality of rolling components (301) arranged along a middle space (k1) in the joint portion (103) to form a contact space (k2) for providing an operating component (C). In a case of deflection, at least one of the plurality of rolling components (301) generates rolling friction with the operating component (C) in the contact space (k2). Therefore, the deflection joint structure (100) may provide more effective support for the operating component (C) with less energy loss when the operating component (C) controls the deflection component (A) to deflect relative to the fixed component (B), and prevent the operating component (C) from fails such as instability buckling.

## Description

### TECHNICAL FIELD

The present disclosure relates to the field of medical equipment technology, and in particular to a deflection joint structure, a stapler, and a medical device.

### BACKGROUND

With the tremendous progress of science and technology, the existing medical devices are undergoing a process of transforming toward automation and even intelligence. Electronic-controlled medical equipment is also increasingly being applied in practical medical operations, such as surgery and medical rehabilitation. Most existing electronic-controlled medical equipment, such as a stapler, requires a deflection structure that may achieve a rotation function to achieve corresponding medical operations. Powers of clamping, stapling, and cutting operations of the stapler is provided by a forward moving of a cutting blade of an electric motor in a handle of the stapler. Due to a small opening in patient's body during a minimally invasive surgery and a complex internal structure of a human body, in order to ensure that a jaw component reaches an affected area smoothly so as to perform surgical operations, it is required that the jaw component of the stapler may achieve a deflection in a certain angle range.

### SUMMARY

An aspect of the present disclosure provides a deflection joint structure for achieving a deflection of a deflection component relative to a fixed component, comprising: a first connecting portion provided on the deflection component; a second connecting portion provided on the fixed component; a joint portion, a part of the joint portion being provided in the first connecting portion, and another part of the joint portion being provided in the second connecting portion, wherein the joint portion comprises: a plurality of rolling components arranged along a middle space in the joint portion so as to form a contact space for providing an operating component, wherein at least in a case of the deflection, at least one of the plurality of rolling components generates a rolling friction with the operating component in the contact space.

According to the embodiments of the present disclosure, the first connecting portion comprises: a deflection block provided above a part region of the joint portion; a deflection seat provided below a part region of the joint portion; and wherein a first setting space is formed between the deflection block and the deflection seat.

According to the embodiments of the present disclosure, the deflection block comprises: a block deflection hole penetrating an end portion of the deflection block away from the deflection component; a block joint hole provided at the end portion close to the deflection component; a block connecting column provided on a side of the end portion opposite to the block deflection hole.

According to the embodiments of the present disclosure, the deflection seat comprises: a seat deflection hole penetrating an end portion of the deflection seat away from the deflection component; a seat joint hole provided at the end portion close to the deflection component; a seat connecting hole provided on a side of the end portion opposite to the seat deflection hole.

According to the embodiments of the present disclosure, the second connecting portion comprises: a block deflection element corresponding to the deflection block and vertically provided on an end surface of an end portion of the fixed component close to the deflection component; a seat deflection element corresponding to the deflection seat and provided symmetrically with the block deflection element on the end surface relative to the end surface; and wherein a second setting space is formed between the block deflection element and the seat deflection element.

According to the embodiments of the present disclosure, the block deflection element comprises: a block deflection column provided in the block deflection hole of the deflection block, so that the block deflection element is located on an outer side of the deflection block.

According to the embodiments of the present disclosure, the seat deflection element comprises: a seat deflection column provided in a seat deflection hole of the deflection seat, so that the seat deflection element is located on an outer side of the deflection seat.

According to the embodiments of the present disclosure, the joint portion comprises: a block guard plate provided on a side of the plurality of rolling components; a seat guard plate provided on the other side of the plurality of rolling components and forming the middle space of the joint portion.

According to the embodiments of the present disclosure, the block guard plate comprises: a first joint column corresponding to a block joint hole of a deflection block of the first connecting portion, wherein the first joint column is provided at an end portion of the block guard plate close to the first connecting portion; a second joint column corresponding to a first defining groove of a block deflection element of the second connecting portion, wherein the second joint column is provided at the other end portion of the block guard plate close to the second connecting portion.

According to the embodiments of the present disclosure, the seat guard plate comprises: a third joint column corresponding to a seat joint hole of a deflection seat of the first connecting portion, wherein the third joint is provided at an end portion of the seat guard plate close to the first connecting portion; a fourth joint column corresponding to a second defining groove of a seat deflection element of the second connecting portion, wherein the fourth joint is provided at the other end portion of the seat guard plate close to the second connecting portion.

According to the embodiments of the present disclosure, the deflection joint structure further includes: a driving element perpendicular to an end surface of an end portion of the fixed component close to the second connecting portion and movably connected to the first connecting portion, so as to drive the deflection component to deflect relative to the fixed component when extending or retracting relative to the second connecting portion.

According to the embodiments of the present disclosure, the deflection joint structure further includes: a driving connection hole forming a penetrating relationship with a block connecting column of a deflection block of the first connecting portion, so that the block connecting column penetrates the driving connection hole, and further into a seat connecting hole of the deflection seat of the first connecting portion after, so as to form a rotating secondary structure between the first connecting portion and the second connecting portion.

According to the embodiments of the present disclosure, the plurality of rolling components comprise: at least one first rolling component corresponding to the rotating secondary structure, provided between the block guard plate and the seat guard plate, and located at an edge of the joint portion; at least three second rolling components cooperated with the at least one first rolling component, provided between the block guard plate and the seat guard plate, and located at the edge of the joint portion; and wherein the at least one first rolling component and the at least three second rolling components form the contact space located in the middle of the joint portion, and the contact space is configured to provide a space for the operating component to pass through and generate rolling friction with the plurality of rolling components in the case of the deflection.

According to the embodiments of the present disclosure, the block guard plate comprises: a first notch provided on the block guard plate and matching with the rotating secondary structure in the case of the deflection.

According to the embodiments of the present disclosure, the seat guard plate comprises: a second notch corresponding to the first notch, provided on the seat guard plate, and matching with the rotating secondary structure in the case of the deflection.

According to the embodiments of the present disclosure, each of the at least one first rolling component comprises: a block rolling element provided on a block rolling column provided on an inner surface of the block guard plate and rotating relative to the block rolling column; a seat rolling element provided on a seat rolling column provided on an inner surface of the seat guard plate and rotating relative to the seat rolling column; and wherein an abdication space is formed between the block rolling element and the seat rolling element, and the abdication space is configured to accommodate the driving element to pass through or stay in the case of the deflection.

According to the embodiments of the present disclosure, each of the at least one second rolling component comprises: at least one middle rolling element provided on at least one corresponding middle rolling column provided on the seat guard plate and rotating relative to the at least one middle rolling column.

According to the embodiments of the present disclosure, the plurality of rolling components are respectively arranged in an arc shape at two side edges of the joint portion, along an extension direction of the operating component in the contact space and on two sides of the contact space.

According to the embodiments of the present disclosure, the plurality of rolling components include two third rolling components and two fourth rolling components. The two third rolling components are provided at an end of the joint portion close to the first connecting portion and arranged on two sides of an end of the contact space; and the two fourth rolling components are provided at the other end of the joint portion close to the second connecting portion and arranged on two sides of the other end of the contact space.

According to an embodiment of the present disclosure, the plurality of rolling components further include two fifth rolling components. The two fifth rolling components are provided in the middle of the joint portion and arranged on two sides of the middle of the contact space; a first spacing is formed between one of the two third rolling components and one of the two fifth rolling components close to the rotating secondary structure; a second spacing is formed between the other of the two fifth rolling components and the other of the two third rolling components, and the first spacing is less than the second spacing.

Another aspect of the present disclosure provides a stapler, comprising: the deflection joint structure as described above; the deflection component connected to the first connecting portion of the deflection joint structure; the fixed component connected to the second connecting portion of the deflection joint structure; the operating component passing the contact space formed between the plurality of rolling components of the deflection joint structure; wherein in the case of the deflection of the deflection component relative to the fixed component, at least one of the plurality of rolling components generates rolling friction with the operating component in the contact space.

Yet another aspect of the present disclosure provides a medical device, comprising the deflection joint structure as described above.

Therefore, by providing a cooperating structure having the operating component and rolling components with rolling friction, the deflection joint structure in the embodiments of the present disclosure may provide more effective support for the operating component with less energy loss when the operating component controls the deflection component to deflect relative to the fixed component, and prevent the operating component from fails such as instability buckling.

### BRIEF DESCRIPTION OF THE DRAWINGS

FIG. 1 schematically shows an exploded composition three-dimensional diagram of a deflection joint structure 100 according to an embodiment of the present disclosure;
FIG. 2A schematically shows a three-dimensional diagram of a deflection block 110 of a first connecting potion 101 according to an embodiment of the present disclosure;
FIG. 2B schematically shows a three-dimensional diagram of a block deflection element 210 of a second connecting potion 102 according to an embodiment of the present disclosure;
FIG. 3 schematically shows a three-dimensional diagram of a deflection joint structure 100 according to an embodiment of the present disclosure;
FIG. 4 schematically shows a cross-sectional view of a deflection joint structure 100 according to an embodiment of the present disclosure;
FIG. 5 schematically shows a top plan view of a deflection joint structure 100 in a state of no bending (without a deflection seat 120, a seat deflection element 220, and a seat guard plate 320) according to an embodiment of the present disclosure;
FIG. 6 schematically shows a three-dimensional diagram of a deflection joint structure 100 (without a seat deflection element 220 and a block deflection element 210) according to an embodiment of the present disclosure;
FIG. 7A schematically shows a top plan view of a deflection joint structure 100 in a state of bending in a direction (without a deflection seat 120, a seat deflection element 220, and a seat guard plate 320) according to an embodiment of the present disclosure;
FIG. 7B schematically shows a top plan view of a deflection joint structure 100 in a state of bending in a direction (with a deflection seat 120, a seat deflection element 220, and a seat guard plate 320) according to an embodiment of the present disclosure;
FIG. 8A schematically shows a top plan view of a deflection joint structure 100 in a state of bending in another direction (without a deflection seat 120, a seat deflection element 220, and a seat guard plate 320) according to an embodiment of the present disclosure;
FIG. 8B schematically shows a top plan view of a deflection joint structure 100 in a state of bending in another direction (with a deflection seat 120, a seat deflection element 220, and a seat guard plate 320) according to an embodiment of the present disclosure;
FIG. 9A schematically shows a three-dimensional diagram of a joint portion 103 from a perspective according to an embodiment of the present disclosure;
FIG. 9B schematically shows a three-dimensional diagram of a joint portion 103 from another perspective according to an embodiment of the present disclosure;
FIG. 10 schematically shows an exploded composition three-dimensional diagram of a joint portion 103 according to an embodiment of the present disclosure;
FIG. 11 schematically shows another exploded composition three-dimensional diagram of a joint portion 103 according to an embodiment of the present disclosure.

### DETAILED DESCRIPTION OF EMBODIMENTS

In order to make purposes, technical solutions, and advantages of the present disclosure clearer, the present disclosure will be further explained in detail in combination with specific embodiments and with reference to the accompanying drawings.

It should be noted that the implementations not shown or described in the accompanying drawings or the specification are all in forms known to those ordinary skilled in the art, which are not explained in detail. In addition, the above definitions of each element and method are not limited to various specific structures, shapes, or methods mentioned in the embodiments, and may be simply modified or replaced by those ordinary skilled in the art.

It should also be noted that the directional terms mentioned in the embodiments, such as "up", "down", "front", "back", "left", "right", etc., are only for reference to directions in the drawings and are not intended to limit the scope of protection of the present disclosure. Throughout the drawings, the same elements are represented by the same or similar reference numerals. Conventional structures or constructions will be omitted since they may cause confusion in the understanding of the present disclosure.

In addition, shapes and sizes of each component in the drawings do not reflect real sizes and proportions, but only represent contents of the embodiments of the present disclosure. Furthermore, in the claims, any reference numeral located between parentheses should not be constructed as a limitation on the claims.

Furthermore, the word "including" does not exclude a presence of elements or steps not listed in the claims. The word "one" or "a" before an element does not exclude an existence of a plurality of such elements.

The use of ordinal numbers such as "first", "second", "third", etc. in the specification and claims for describing corresponding elements does not imply that the elements have any ordinal numbers, nor does it represent the order of one element and another element or the order of a manufacturing method. The use of these ordinal numbers is only to enable an element with a certain name to be clearly distinguished from another element with the same name.

Those skilled in the art may understand that modules in a device in an embodiment may be adaptively changed and provided in one or more devices different from the embodiment. The modules, units, or components in an embodiment may be combined into one module, unit, or component. In addition, the modules, units, or components in the embodiment may be divided into a plurality of sub-modules, sub-units, or sub-components. Except for at least some of such features and/or processes or units being mutually exclusive, any combination may be used to combine all features disclosed in the specification (including accompanying claims, abstracts, and drawings), as well as all processes or units of any method or device thus disclosed. Unless otherwise explicitly stated, each feature disclosed in the specification (including accompanying claims, abstracts, and drawings) may be replaced by alternative features that provide the same, equivalent, or similar purposes. Moreover, in the unit claims listing several apparatuses, several of these apparatuses may be specifically embodied through a same hardware item.

Similarly, it should be understood that in order to streamline the present disclosure and assist in understanding one or more aspects of the present disclosure, in the descriptions of exemplary embodiments of the present disclosure above, various features of the present disclosure are sometimes grouped together into a single embodiment, drawing, or description thereof. However, such disclosed method should not be interpreted as reflecting an intention that the claimed protection of the present disclosure has more features than those explicitly recorded in each claim. More precisely, as reflected in the following claims, the disclosed aspects are less than all features of the previously disclosed single embodiments. Therefore, the claims following the specific implementations are explicitly incorporated into the specific implementations, and each claim itself serves as a separate embodiment of the present disclosure.

The stapler is commonly used in minimally invasive surgeries. During surgery, a jaw component of the stapler directly performing stapling and cutting operations passes through an internal hole of a puncture device fixed on a patient's body to reach an affected part, so as to perform clamping, stapling, and cutting operations on the patient's tissue. After the jaw component of the stapler is deflected, it still needs to meet requirements of clamping, stapling and cutting operations. Therefore, a cutting blade of the stapler generally has a flexible handle. It is required that the handle of the cutting knife can still transmit force along an axial direction of the handle after bending with a deflection joint.

In case that the handle of the cutting knife continues to transmit the axial force after bending and deforming, if there is no reliable protective wall support, the axial force required for the stapling and cutting operations may easily cause fails of the cutting blade such as instability buckling. After the handle of the cutting blade buckles or breaks, a blade head loses power and cannot move, and the jaw component cannot open, causing the tissue to be unable to be extracted from the jaw, seriously damaging the patient's tissue. Moreover, the failed handle may impact the patient's tissue, causing secondary injury. After the above medical accidents occur, open surgery is required to remedy, which significantly increases a difficulty and a risk of the surgery.

Specifically, the deflection joint of the stapler in the prior art generally uses a rigid integrated protective wall or a flexible shrapnel protective wall to support the handle of the cutting blade at the deflection joint. Two ends of the rigid integrated protective wall are respectively hinged with fixed ends of the jaw component and the deflection joint, while two ends of the flexible shrapnel protective wall are completely fixed with the fixed ends of the jaw component and the deflection joint respectively, achieving a dynamic deflection of the protective wall during a jaw deflection process. However, the existing solutions mentioned above involve a sliding friction between the protective wall and the handle of the cutting blade when stapling and cutting operations are performed in case that the jaw component of the stapler deflects. During the deflection process of the flexible shrapnel protective wall, a bending stress of the shrapnel is high, and a support force that may be provided for the handle of the cutting blade is relatively small. Moreover, the use of sliding friction results in a relatively large frictional force between the protective wall and the handle of the cutting blade. Therefore, when the stapler in the prior art is used for stapling and cutting operations, in order to overcome the relatively large frictional force between the handle and the protective wall in the deflection structure, a greater axial force needs to be provided for the cutting blade at the handle end, which mainly produces the following problems: (1) the flexible handle of the existing cutting blade has a greater aspect ratio, which makes it more prone to instability failure when subjected to a large axial force, and a safety thereof is not high; (2) power transmission components from a power component to the handle need to withstand greater loads and have higher strength requirements, which may increase a weight of the stapler or increase a production cost; (3) an electric stapler requires a higher power motor, while a manual stapler requires operators to provide greater operating force, both of which increase an energy consumption.

To solve at least one of the technical problems existing in the deflection function of medical equipment devices such as staplers in the prior art, the present disclosure provides a deflection joint structure, a stapler, and a medical device, aiming to provide a deflection joint structure with a rolling friction cutting blade protective wall that may be applied to medical devices such as staplers.

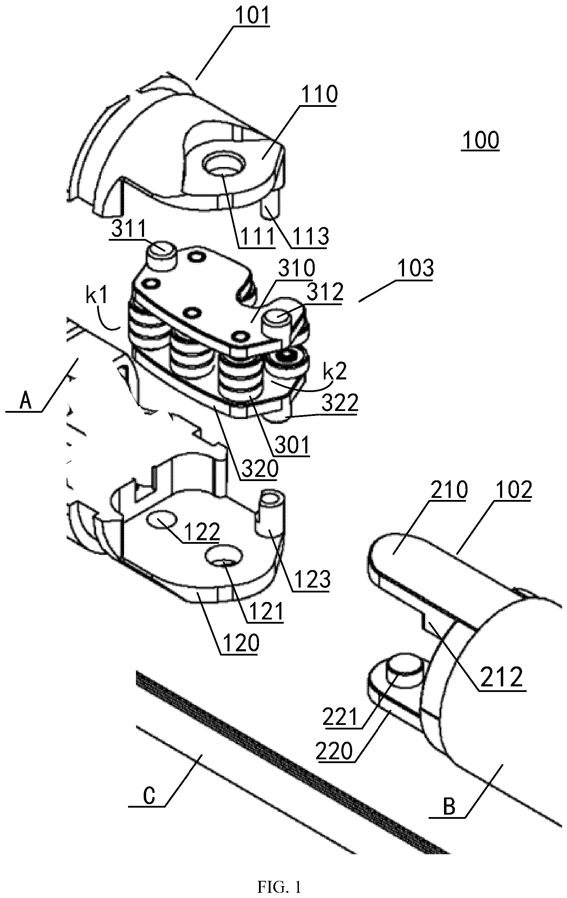

As shown in FIG. 1, an aspect of the present disclosure provides a deflection joint structure 100 achieving a deflection of a deflection component A relative to a fixed component B. The deflection joint structure 100 includes a first connecting portion 101, a second connecting portion 102, and a joint portion 103.

The first connecting portion 101 is provided on the deflection component A.

The second connecting portion 102 is provided on the fixed component B.

A part of the joint portion 103 is provided in the first connecting portion 101, and the other part of the joint portion 103 is provided in the second connecting portion 102. The joint portion 103 includes a plurality of rolling components 301. The plurality of rolling components 301 are arranged along a middle space k1 in the joint portion 103 to form a contact space k2 for providing an operating component C. In a case of deflection, at least one of the plurality of rolling components 301 generates rolling friction with the operating component C in the contact space k2.

The deflection component A may serve as a main operating contact portion used to corporate with the operating component C so as to achieve an operation of the operating component. The fixed component B may serve as a main operating control portion used to control a deflection of the deflection component A and an operation of the operating component C. The deflection component A performs a deflection action relative to the fixed component B, and the operating component C may pass through the deflection component A and the fixed component B, so that when the deflection component A deflects relative to the fixed component B, a targeted operation may be achieved, while an installation of the operating component C may also be ensured.

The first connecting portion 101 is inter-configured with the deflection component A. Specifically, a part or a whole of the first connecting portion 101 is fixedly connected to the deflection component A through a fixed connection method. The second connecting portion 102 is inter-configured with the fixed component B. Specifically, a part or a whole of the second connecting portion 102 is fixedly connected to the fixed component B through a fixed connection method. The first connecting portion 101 and the second connecting portion 102 are respectively fixed on the corresponding deflection component A and fixed component B.

The joint portion 103 is located between the deflection component A and the fixed component B, and in a space formed between the first connecting portion 101 and the second connecting portion 102. A part of the joint portion 103 close to the deflection component A is located in an internal space of the first connecting portion 101. A part of the joint portion 103 close to the fixed component B is located in an internal space of the second connecting portion 102.

The middle space k1 is formed in the middle of the joint portion 103. The middle space k1 is used to provide the plurality of rolling components 301 and provide positions for the rolling components 301. The rolling component 301 may generally be a spherical or cylindrical structure, which may spin around a fixed axis. In the joint portion 103, each rolling component 301 may perform a movably spin action relative to the joint portion 103. The rolling component 301 may specifically be a bearing structure. With the help of the arrangement of the plurality of rolling components 301 in the middle space k1 of the joint portion 103, the contact space k2 through which the above-mentioned operating component C may pass may be formed in the middle space k1 of the joint portion 103. That is, the operating component C extending from the fixed component B may pass through the contact space k2 of the joint portion 103 and extend into the deflection component A. The operating component C may be a strip flexible component, such as a flexible cutting blade of the stapler, etc.

When the deflection component A deflects relative to the fixed component B, the flexible operating component C may be driven by the deflection component A to undergo flexible bending in the contact space k2 of the joint portion 103. A bending part of the operating component C may be in contact with the corresponding rolling component 301 in the contact space k2 in a case of a certain deflection angle. Since the rolling component 301 may spin relative to the joint portion 103, an external force may be applied to the rolling component 301 when the bending part of the operating component C is in contact with the rolling component 301, so that the contacting rolling component 301 spins.

In other words, compared to the sliding friction design between the operating component C and the joint portion in the prior art, the above-mentioned deflection joint structure 100 of the embodiment of the present disclosure utilizes the design of the rolling component 301 of the joint portion 103 to achieve rolling friction between the bending part of the operating component C and the rolling components 301 in contact with each other. In this way, not only the operating component C is effectively supported, but the contact friction between the operating component C and the joint portion 103 is greatly reduced. Therefore, during the operation process, a driving force required for the operating component C may be greatly reduced, a reliable and stable structural support may be provided for the operating component C, a stability of the operating component C may be improved, and the instability failure of the operating component C may be avoided. Furthermore, a structural safety and stability of the deflection component A, the fixed component B, the operating component C, and the joint portion 103 may be enhanced, the requirement for a power system may be further reduced, and an overall energy efficiency may be improved.

Therefore, by providing a cooperating structure having the operating component C and rolling components 301 of the joint portion 103 with rolling friction, the deflection joint structure 100 in the embodiment of the present disclosure may provide a more effective support for the operating component with less energy loss when the operating component controls the deflection component A to deflect relative to the fixed component B, and prevent the operating component from fails such as instability buckling. An overall structure is safer and more controllable with flexible and free rotation, achieving further integration of the joint portion 103 and facilitating a deflection with a small turning radius.

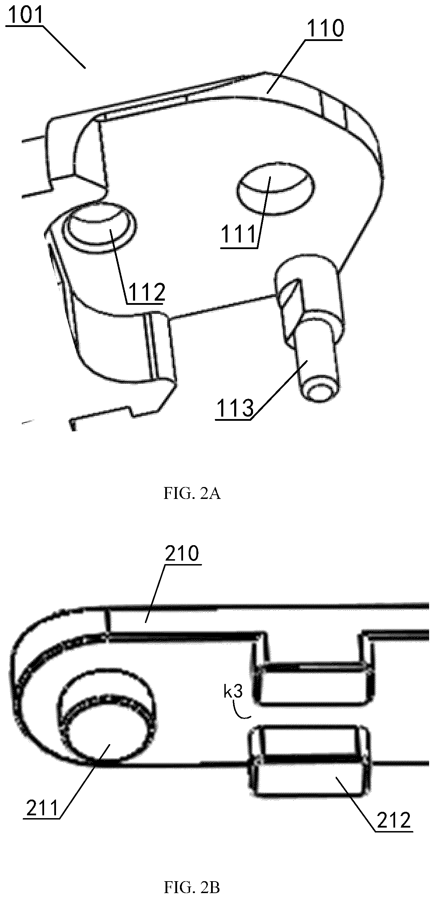

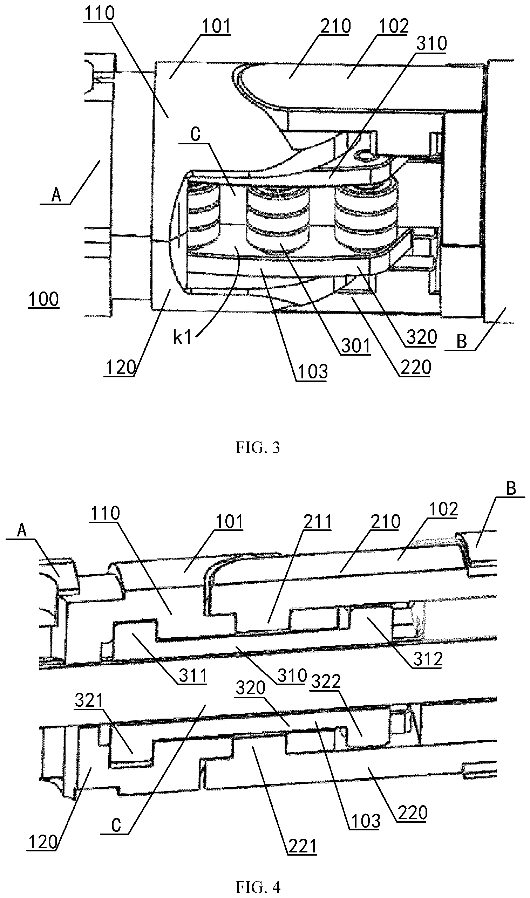

As shown in FIG. 1 to FIG. 8B, according to an embodiment of the present disclosure, the first connecting portion 101 includes a deflection block 110 and a deflection seat 120.

The deflection block 110 is provided above a part region of the joint portion 103.

A deflection seat 120 is provided below a part region of the joint portion 103.

A first setting space is formed between the deflection block 110 and the deflection seat 120.

The deflection block 110 is provided above a part of the joint portion 103. The deflection seat 120, corresponding to the deflection block 110, is provided below another part of the joint portion 103. It should be understood that the deflection block 110 and the deflection seat 120, as main structural components of the first connecting portion 101, may be symmetrically arranged on two sides of a structural part of the joint portion 103 close to the deflection component A. That is, a part of the joint portion 103 is located in the first setting space formed between the deflection block 110 and the deflection seat 120.

In this way, a stable connection between the joint portion 103 and the first connecting portion 101 may be ensured while a structural integration between the joint portion 103 and the first connecting portion 101 may also be ensured.

As shown in FIG. 1 to FIG. 8B, according to an embodiment of the present disclosure, the deflection block 110 includes a block deflection hole 111, a block joint hole 112, and a block connecting column 113.

The block deflection hole 111 penetrates an end portion of the deflection block 110 away from the deflection component A.

The block joint hole 112 is provided at the end portion close to the deflection component A.

The block connecting column 113 is provided on a side of the end portion opposite to the block deflection hole 111.

The block deflection hole 111 may generally be a circular through hole. located at an end portion of the deflection block 110 close to the fixed component B and may penetrate the deflection block 110 for an movable connection with a block deflection element 210 of the second connecting portion 102 as described below. In this way, the movable connection between the first connecting portion 101 and the second connecting portion 102 on a side of the joint portion 103 may be achieved.

The block joint hole 112 may generally be a circular hole located on a part of the deflection block 110 close to the deflection component A relative to the block deflection hole 111, and located above an end portion of the joint portion 103 for an movable connection with a first joint column 311 of the joint portion 103 as described below. In this way, the movable connection between the first connecting portion 101 and one side of the joint portion 103 may be achieved.

The block connecting column 113 is provided at an edge of the deflection block 110 on a side of the block deflection hole 111. The block connecting column 113 protrudes outward from an inner surface of the deflection block 110 toward the joint portion 103. The block connecting column 113 may have a column base and a column tip that are used to cooperate with a seat connecting hole 123 of the deflection seat 120 described below so as to achieve a positioning connection for a driving element 104.

Therefore, the connection between the joint portion 103 and the first connecting portion 101 as well as the connection between the second connecting portion 102 and the first connecting portion 101 on a side of the joint portion 103 may be achieved by using the deflection block 110, so as to ensure a basic connection structure of the first connecting portion 101, the second connecting portion 102, and the joint portion 103.

As shown in FIG. 1 to FIG. 8B, according to an embodiment of the present disclosure, the deflection seat 120 includes a seat deflection hole 121, a seat joint hole 122, and a seat connecting hole 123.

The seat deflection hole 121 penetrates an end portion of the deflection seat 120 away from the deflection component A.

The seat joint hole 122 is provided on the end portion close to the deflection component A.

The seat connecting hole 123 is provided on a side of the end portion opposite to the seat deflection hole 121.

The seat deflection hole 121 may generally be a circular through hole. located at an end portion of the deflection seat 120 close to the fixed component B and may penetrate the deflection seat 120, for an movable connection with the seat deflection element 220 of the second connecting portion 102 as described below. In this way, an movable connection between the first connecting portion 101 and the second connecting portion 102 on the other side of the joint portion 103 may be achieved.

The seat joint hole 122 may generally be a circular hole located on a part of the deflection seat 120 close to the deflection component A relative to the seat deflection hole 121, and located below an end portion of the joint portion 103 for a mutual movable connection with a second joint column 312 of the joint portion 103 as described below. In this way, an movable connection between the first connecting portion 101 and the other side of the joint portion 103 may be achieved.

The seat connecting hole 123 is provided at an edge of the deflection seat 120 on a side of the seat deflection hole 121. The seat connecting hole 123 is provided from an inner surface of a fixed hole 110 toward the joint portion 103. A seat joint hole 123 may be a column hole, i.e. an external protruding column structure with a blank space in the middle, which is used to cooperate with the block connecting column 113 of the deflection block 110 described above so as to achieve the positioning connection for the driving element 104.

Therefore, the connection between the joint portion 103 and the first connecting portion 101 as well as the connection between the second connecting portion 102 and the first connecting portion 101 on the other side of the joint portion 103 may be achieved by using the deflection seat 120, so as to further ensure the basic connection structure of the first connecting portion 101, the second connecting portion 102, and the joint portion 103.

As shown in FIG. 1 to FIG. 8B, according to an embodiment of the present disclosure, the second connecting portion 102 includes a block deflection element 210 and a seat deflection element 220.

The block deflection element 210 corresponds to the deflection block 110 and is vertically provided on an end surface of an end portion of the fixed component B close to the deflection component A.

The seat deflection element 220 corresponds to the deflection seat 120 and is provided symmetrically with the block deflection element 210 on the end surface relative to the end surface.

A second setting space is formed between the block deflection element 210 and the seat deflection element 220.

The block deflection element 210 and the seat deflection element 220 with symmetrical locations are vertically provided on the end surface of the fixed component B toward the deflection component A. The block deflection element 210 and the seat deflection element 220 are parallel to each other and protrude perpendicular to the end surface toward the deflection component A. The block deflection element 210 is provided above another part of the joint portion 103 relative to the deflection block 110, while the seat deflection element 220, corresponding to the block deflection element 210, is provided below the another part of the joint portion 103. It should be understood that the block deflection element 210 and the seat deflection element 220, as main structural components of the second connecting portion 102, may be symmetrically arranged on two sides of a structural part of the joint portion 103 close to the fixed component B. That is, the another part of the joint portion 103 is located in the second setting space formed between the block deflection element 210 and the seat deflection element 220.

In this way, a stable connection between the joint portion 103 and the second connecting portion 102 may be ensured while a structural integration between the joint portion 103 and the second connecting portion 102 may also be ensured.

The block deflection element 210 is connected to the deflection block 110 at the same time, and the seat deflection element 220 is connected to the deflection seat 120 at the same time, thereby achieving a connection between the first connecting portion 101 and the second connecting portion 102. Moreover, the block deflection element 210 and the deflection block 110 may relatively deflect, and the seat deflection element 220 and the deflection seat 120 may relatively deflect. The joint portion 103 and the first connecting portion 101 may relatively deflect, and the joint portion 103 and the second connecting portion 102 may also relatively deflect. In this way, a deflection effect of the deflection joint of the joint portion 103 may be achieved.

As shown in FIG. 1 to FIG. 8B, according to an embodiment of the present disclosure, the block deflection element 210 includes a block deflection column 211 provided in the block deflection hole 111 of the deflection block 110, so that the block deflection element 210 is located on an outer side of the deflection block 110.

The block deflection column 211 is generally a cylindrical structure that matches a shape of the block deflection hole 111 of the deflection block 110. The block deflection column 211 may be inserted into the block deflection hole 111 to achieve the connection between the block deflection element 210 and the deflection block 110. At the same time, when the block deflection column 211 rotates relative to the block deflection hole 111, a relative rotation between the block deflection element 210 and the deflection block 110 may be achieved.

The block deflection column 211 protrudes outward from an inner surface of the block deflection element 210 toward the seat deflection element 220 so as to form a cylindrical structure. Therefore, the block deflection column 211 and the block deflection hole 111 are connected and matched, so that the block deflection element 210 is located on the outer side of the deflection block 110. An abdication structure for accommodating the block deflection element 210 is provided on an outer surface of the deflection block 110 toward the block deflection element 210, so that the block deflection element 210 may rotate in a space of the abdication structure, thereby satisfying a miniaturization and an integration of the structural volume.

Therefore, the connection between the first connecting portion 101 and the second connecting portion 102 on a side of the joint portion may be achieved by using the block deflection column 211 and the block deflection hole 111, and the two may rotate relative to each other so as to achieve the joint function at the same time.

According to an embodiment of the present disclosure, the seat deflection element 220 includes a seat deflection column 221 provided in the seat deflection hole 121 of the deflection seat 120, so that the seat deflection element 220 is located on an outer side of the deflection seat 120.

The seat deflection column 221 is generally a cylindrical structure that matches a shape of the seat deflection hole 121 of the deflection seat 120. The seat deflection column 221 may be inserted into the seat deflection hole 121 to achieve the connection between the seat deflection element 220 and the deflection seat 120. At the same time, when the seat deflection column 221 rotates relative to the seat deflection hole 121, a relative rotation between the seat deflection element 220 and the deflection seat 120 may be achieved.

The seat deflection column 221 protrudes outward from an inner surface of the seat deflection element 220 toward the block deflection element 210, to form a cylindrical structure. Therefore, the seat deflection column 221 and the seat deflection hole 121 are connected and matched, so that the seat deflection element 220 is located on the outer side of the deflection seat 120. An abdication structure for accommodating the seat deflection element 220 is provided on an outer surface of the deflection seat 120 toward the seat deflection element 220, so that the seat deflection element 220 may rotate in a space of the abdication structure, thereby satisfying the miniaturization and the integration of the structural volume.

Therefore, the connection between the first connecting portion 101 and the second connecting portion 102 on the other side of the joint portion may be achieved by using the seat deflection column 221 and the seat deflection hole 121. At the same time, the two may cooperate with the above-mentioned block deflection column 211 and block deflection hole 111 such that the fixed component B may be rotate relative to the deflection component A, so as to achieve the joint function.

It should be noted that an overall structure of the deflection block 110 and an overall structure of the deflection seat 120 may be symmetrically arranged with each other, and sizes and shapes of the deflection block 110 and the deflection seat 120 may be substantially consistent. Similarly, an overall structure of the block deflection element 210 and an overall structure of the seat deflection element 220 may also be symmetrically arranged with each other, and sizes and shapes of the block deflection element 210 and the seat deflection element 220 may also be substantially consistent. The first connecting portion 101 may be fixedly connected to the deflection component A, and may not generate any movable action relative to the deflection component A generally. The second connecting portion 102 may be fixedly connected to the fixed component B, and may not generate any movable action relative to the fixed component B generally.

As shown in FIG. 1 to FIG. 11, according to an embodiment of the present disclosure, the joint portion 103 includes a block guard plate 310 and a seat guard plate 320.

The block guard plate 310 is provided on a side of the plurality of rolling components 301.

The seat guard plate 320 is provided on the other side of the plurality of rolling components 301, forming the middle space k1 of the joint portion 103.

The block guard plate 310 is a plate-like structure. A part of an outer surface of the block guard plate faces the deflection block 110, while another part of the outer surface of the block guard plate 310 faces the block deflection element 210 and is provided in the setting space formed by the first connecting portion 101 and the second connecting portion 102 (i.e. a composition of the first setting space and the second setting space). An inner surface of the block guard plate 310 faces the plurality of rolling components 301, serving as a side edge of the middle space k1 where the rolling components 301 are located.

The seat guard plate 320 is a plate-like structure. A part of an outer surface of the seat guard plate 320 faces the deflection seat 120, while another part of the outer surface of the seat guard plate 320 faces the seat deflection element 220 and is provided in the setting space formed by the first connecting portion 101 and the second connecting portion 102 (i.e. the composition of the first setting space and the second setting space). An inner surface of the seat guard plate 320 is provided towards the plurality of rolling components 301, serving as the other side edge of the middle space k1 where the rolling components 301 are located.

Shapes of the block guard plate 310 and the seat guard plate 320 may be consistent with each other, or there may also be some differences. Generally, edges of the block guard plate 310 and the seat guard plate 320 may adopt curved designs for enhancement. The middle space k1 formed between the block guard plate 310 and the seat guard plate 320 may be used to provide the rolling components 301. The rolling components 301 may be positioned and distributed in the middle space k1 by providing corresponding installation structures on the inner surfaces of the block guard plate 310 and the seat guard plate 320.

In this way, the block guard plate 310 and the seat guard plate 320 may provide a setting position and a setting space for the rolling components 301 in the joint portion 103, so as to avoid affecting the rolling components 301 when the deflection component A deflects relative to the fixed component B, and ensure that the rolling friction of stable contact generated between the rolling components 301 and the operating component C passing therethrough.

As shown in FIG. 1 to FIG. 11, according to an embodiment of the present disclosure, the block guard plate 310 includes a first joint column 311 and a second joint column 312.

The first joint column 311 corresponds to the block joint hole 112 of the deflection block 110 of the first connecting portion 101 and is provided at an end portion of the block guard plate 310 close to the first connecting portion 101.

The second joint column 312 corresponds to a first defining groove of the block deflection element 210 of the second connecting portion 102 and is provided at the other end portion of the block guard plate 310 close to the second connecting portion 102.

The first joint column 311 may be a cylindrical structure protruding outward from an outer surface of the block guard plate 310, and may be located at an end of the block guard plate 310 close to the deflection component A. The first joint column 311 may match with the block joint hole 112, so that the first joint column 311 may rotate relative to the block joint hole 112, thereby achieving the relative rotation of the joint portion 103 relative to the first connecting portion 101 on a side of the joint portion.

The second joint column 312 may be a cylindrical structure protruding outward from the outer surface of the block guard plate 310, and may be located at the other end of the block guard plate 310 close to the fixed component B. The second joint column 312 may match with the first defining groove formed on the inner surface of the block deflection element 210, so that the second joint column 312 may rotate relative to the first defining groove, thereby achieving the relative rotation of the joint portion 103 relative to the second connecting portion 102 on a side of the joint portion.

As shown in FIGS. 1, 2B, and 4, the first defining groove k3 is located on the inner surface of the block deflection element 210, and is formed by clamping two protruding structures 212 that respectively protrude outward from edges of two sides of the inner surface of the block deflection element 210 toward the seat deflection element 220 and are parallel to each other. Due to a limit setting of the first joint column 311, the first defining groove k3 only needs to limit two sides of the second joint column 312.

In this way, the movable connection between the first connecting portion 101 and the joint portion 103, as well as the movable connection between a second joint portion 102 and the joint portion 103 on a side of the joint portion 103, may be achieved. The structural design is simple and achieves the effect of connection limit but not fixation, allowing for pairwise rotation among the joint portion 103, the first connecting portion 101, and the second connecting portion 102.

As shown in FIG. 1 to FIG. 11, according to an embodiment of the present disclosure, the seat guard plate 320 includes a third joint column 321 and a fourth joint column 322.

The third joint column 321 corresponds to the seat joint hole 122 of the deflection seat 120 of the first connecting portion 101 and is provided at an end portion of the seat guard plate 320 close to the first connecting portion 101.

The fourth joint column 322 corresponds to a second defining groove of the seat deflection element 220 of the second connecting portion 102 and is provided at the other end portion of the seat guard plate 320 close to the second connecting portion 102.

The third joint column 321 may be a cylindrical structure protruding outward from the outer surface of the seat guard plate 310, and may be located at an end of the seat guard plate 320 close to the deflection component A. The third joint column 321 may match with the seat joint hole 122, so that the third joint column 321 may rotate relative to the seat joint hole 122, thereby achieving the relative rotation of the joint portion 103 relative to the first connecting portion 101 on the other side of the joint portion.

The fourth joint column 322 may be a cylindrical structure protruding outward from the outer surface of the seat guard plate 320, and may be located at the other end of the seat guard plate 320 close to the fixed component B. The fourth joint column 322 may match with the second defining groove formed on the inner surface of the seat deflection element 220, so that the fourth joint column 322 may rotate relative to the second defining groove, thereby achieving the relative rotation of the joint portion 103 relative to the second connecting portion 102 on the other side of the joint portion.

As shown in FIGS. 1, 2B and 4, the structural settings of the second defining groove and the first defining groove k3 are substantially the same. The second defining groove may be located on the inner surface of the seat deflection element 220, and is formed by clamping two protruding structures that respectively protrude outward from edges of two sides of the inner surface of the seat deflection element 220 toward the block deflection element 210 and are parallel to each other. Due to a limit setting of the third joint column 321, the second defining groove only needs to limit two sides of the fourth joint column 322.

In this way, the movable connection between the first connecting portion 101 and the joint portion 103, as well as the movable connection between the second joint portion 102 and the joint portion 103 on the other side of the joint portion 103, may be achieved. The structural design is simple and achieves the effect of connection limit but not fixation, allowing for pairwise rotation among the joint portion 103, the first connecting portion 101, and the second connecting portion 102.

Therefore, the joint portion 103 may be movably connected to the space formed between the first connecting portion 101 and the second connecting portion 102, achieving a highly integrated structure of the three, which is conducive to the miniaturization of the overall structure of the joint portion 103. In addition, the pairwise rotation effect that may be achieved among the joint portion 103, the first connecting portion 101, and the second connecting portion 102 is ensured.

As shown in FIG. 1 to FIG. 11, according to an embodiment of the present disclosure, the deflection joint structure 100 further includes a driving element 104 perpendicular to an end surface of an end portion of the fixed component B close to the second connecting portion 102 and movably connected to the first connecting portion 101, so as to drive the deflection component A to deflect relative to the fixed component B when extending or retracting relative to the second connecting portion 102.

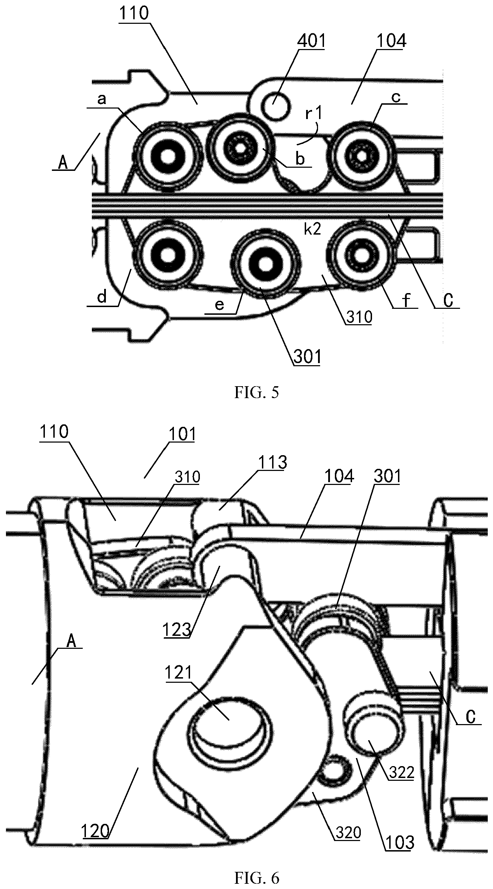

As shown in FIG. 5 to FIG. 8B, the driving element 104 may be a strip plate-like structure, protruding from a space between the block deflection element 210 and the seat deflection element 220, and may be parallel to the block deflection element 210 and the seat deflection element 220. The driving element 104 may pass through the end surface of the fixed component B for providing the block deflection element 210 and the seat deflection element 220, extending or retracting relative to the end surface. That is, the driving element 104 may movably pass through the end surface of the fixed component B and perform a telescopic action relative to the end surface toward the deflection component A. A structure of the driving element 104 in the fixed component B may be connected to a driving apparatus which may be used for outputting a driving force for a stepper motor, etc. When the driving apparatus outputs the driving force to the driving element 104, an end surface of the driving element 104 relative to the fixed component B may extend or retract toward the deflection component A.

The driving element 104 may be movably connected to the first connecting portion 101 and may maintain a relative rotation between the driving element 104 and the first connecting portion 101. In this way, when the driving element 104 extends toward the deflection component A, the deflection component A may deflect relative to the fixed component B in a deflection direction. When the driving element 104 retracts toward the fixed component B, the deflection component A may deflect relative to the fixed component B in a deflection direction opposite to the above-mentioned deflection direction. Generally, when an angle between the deflection component A and the fixed component B is 180°, it may indicate that the driving element 104 is in an initial state.

The driving element 104 may provide a deflection drive for the deflection of the deflection component A, and may also control a direction of the deflection, thereby ensuring that the deflection control of the deflection joint structure 100 is more stable, controllable, and safer, and achieving the cooperation with the first connecting portion 101, which is conducive to ensuring the stability and reliability of the joint portion 103.

As shown in FIG. 1 to FIG. 11, according to an embodiment of the present disclosure, the driving element 104 further includes a driving connection hole 401 forming a penetrating relationship with the block connecting column 113 of the deflection block 110 of the first connecting portion 101, so that the block connecting column 113 penetrates the driving connection hole 401 and further into the seat connecting hole 123 of the deflection seat 120 of the first connecting portion 101 so as to form a rotating secondary structure between the first connecting portion 101 and the second connecting portion 102.

As shown in FIG. 5 to FIG. 8B, to ensure a more stable and reliable movable connection between the first connecting portion 101 and the driving element 104, the driving connection hole 401 is formed by penetrating an end portion of the driving element 104 toward the deflection component A. The driving connection hole 401 may be a circular through hole. The block connecting column 113 provided on the inner surface of the deflection block 110 may directly penetrate the driving connection hole 401 and insert into the seat connecting hole 123 provided on the inner surface of the deflection seat 120, thereby achieving the movable connection between the first connecting portion 101 and the driving element 104.

A column base or a bottom part of the column base of the connection-completed block connecting column 113 may be located outside the driving connection hole 401. The column tip of the connection-completed block connecting column 113 penetrates the driving connection hole 401 and inserts into the seat connecting hole 123. The seat connecting hole 123 is a cylindrical hole with a shape of cylinder, and the cylinder or a bottom part of the cylinder may be located outside the driving connection hole 401. In this way, the driving element 104 may be limited in a fixed position between the deflection block 110 and the deflection seat 120. Due to a fixed connection between the block connecting column 113 and the seat connecting hole 123 after the insertion connection is completed, the driving element 104 may rotate relative to the connecting structure through the driving connection hole 401.

In this way, the rotational connection between the driving element 104 and the first connecting portion 101 is ensured, so that the driving element 104 may achieve a deflection control on the deflection component A in at least two directions by entension and contraction relative to the fixed component B, forming a rotating secondary structure. When the first connecting portion 101 may generate a rotational action relative to the deflection component A (i.e., a rotational connection between the first connecting portion 101 and the deflection component A), and/or the second connecting portion 102 may also generate a rotational action relative to the fixed component B (i.e., a rotational connection between the second connecting portion 102 and the fixed component B), thus a deflection in other directions may be achieved.

As shown in FIG. 5 to FIG. 11, according to an embodiment of the present disclosure, the plurality of rolling components 301 include at least one first rolling component and at least three second rolling components.

The at least one first rolling component corresponds to the rotating secondary structure is provided between the block guard plate 310 and the seat guard plate 320, and is located at an edge of the joint portion 103.

The at least three second rolling components cooperated with the at least one first rolling component are provided between the block guard plate 310 and the seat guard plate 320, and are located at the edge of the joint portion 103.

The at least one first rolling component and the at least three second rolling components form the contact space k2 located in the middle of the joint portion 103, and the contact space k2 is used to provide a space for the operating component C to pass through and generate rolling friction with the plurality of rolling components 301 in the case of the deflection.

The rolling components 301 may be arranged along the edge of the joint portion 103 to form the contact space k2 in the middle space k1 of the joint portion 103. As shown in FIGS. 5, 7A, and 8A, the first rolling components b and c correspond to the rotating secondary structure composed of the aforementioned driving element 104 and are provided in the joint portion 103. The second rolling components a, d, e, and f may be distributed and arranged in the joint portion 103 relative to the contact space k2 and the first rolling components b and c.

As shown in FIG. 5, in the initial state, the angle between the deflection component A and the fixed component B is 180°, which means that the two do not deflect relative to each other, and a length of the driving element 104 extending relative to the fixed component B is constant. In this case, the operating component C enters the deflection component A from the fixed component B through the contact space k2, and may not contact the first rolling components b and c, as well as the second rolling components a, d, e, and f.

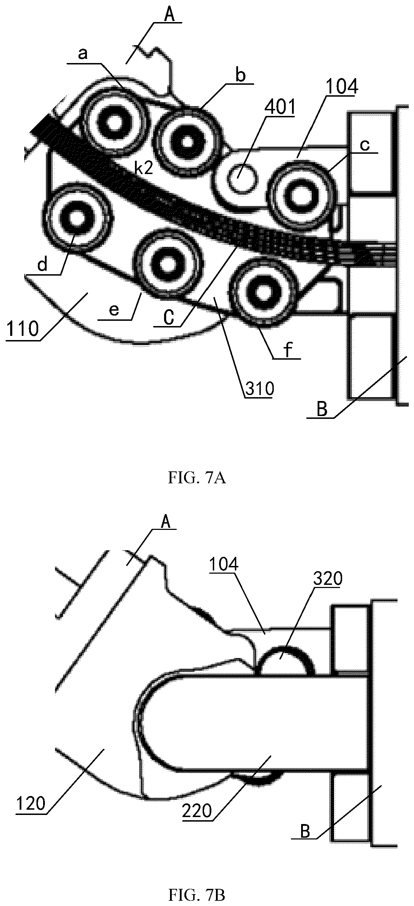

As shown in FIG. 7A, during the deflection process in a certain direction, when the driving element 104 retracts relative to the fixed component B, since a connection position of the driving element 104 and the first connecting portion 101 is located on a side of the operating component C, the driving element 104 may drive the deflection component A to deflect in a deflection direction toward a side of the operating component C through the first connecting portion 101. In this case, the operating component C enters the deflection component A from the fixed component B through the contact space k2 and is driven by the deflection component A to bend, allowing the operating component C to contact at least one of the second rolling components a, d, e, and f. Since each rolling component may undergo positioning rotation, i.e. fixed in a fixed position of the joint portion 103, and may undergo spin rotation, a frictional force between the operating component C and the rolling component in contact is a rolling frictional force rather than a sliding frictional force.

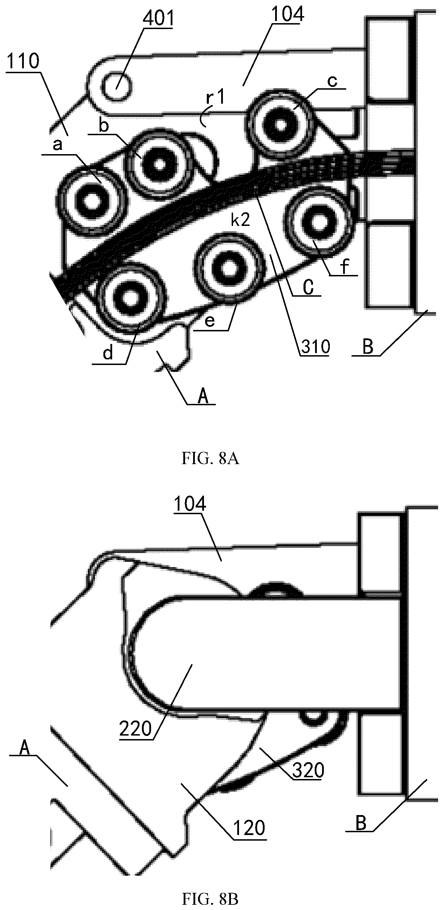

As shown in FIG. 8A, during the deflection process in another direction, when the driving element 104 extends relative to the fixed component B, since the connection position of the driving element 104 and the first connecting portion 101 is located on a side of the operating component C, the driving element 104 may drive the deflection component A to deflect in a deflection direction toward the other side of the operating component C through the first connecting portion 101. In this case, the operating component C enters the deflection component A from the fixed component B through the contact space k2 and is driven by the deflection component A to bend, allowing the operating component C to contact at least one of the second rolling components a and d and the first rolling components b and c. Since each rolling component may undergo positioning rotation, i.e. fixed in a fixed position of the joint portion 103, and may undergo spin rotation, the frictional force between the operating component C and the rolling component in contact is the rolling frictional force, rather than the sliding frictional force.

In other words, with the design of the rolling component 301 in the joint portion 103 mentioned above, a rolling contact may be formed between the operating component C and the rolling component 301 in contact. Compared with the sliding contact in traditional technologies, the contact friction between the two may be greatly reduced, further reducing the driving force required for the operating component C, avoiding the instability failure of the operating component C, and providing a reliable and stable structural support for the operating component C, thereby achieving a more stable and safer deflection joint structure.

As shown in FIG. 5 to FIG. 11, according to an embodiment of the present disclosure, the block guard plate 310 includes a first notch r1 provided on the block guard plate 310 and matching with the rotating secondary structure in the case of deflection. The seat guard plate 320 includes a second notch r2 corresponding to the first notch r1, provided on the seat guard plate 320, and matching with the rotating secondary structure in the case of deflection.

An abdication notch is formed in the joint portion 103 through a corporation of the first notch r1 and the second notch r2. As shown in FIGS. 5, 7A, 8A, and 11, when the deflection component A deflects relative to the fixed component B and the deflection direction of the deflection component A is a side of the operating component C toward the abdication notch (as shown in FIG. 7A), the rotating secondary structure between the driving element 104 and the first connecting portion 101 may slide into the abdication notch along an arc-shaped opening of the abdication notch. In contrast, with the deflection state shown in FIG. 7A as a starting state, when the deflection component A deflects relative to the fixed component B and the deflection direction of the deflection component A is a side of the operating component C away from the abdication notch (as shown in FIG. 8A), the rotating secondary structure between the driving element 104 and the first connecting portion may slide out of the abdication notch and deflect in an opposite direction, causing the driving element 104 to move away from the abdication notch.

Therefore, an overall volume of the deflection joint structure 100 may be greatly reduced while further ensuring the stable deflection, which is conducive to the miniaturization and integration of the structure.

As shown in FIG. 5 to FIG. 11, according to an embodiment of the present disclosure, each of the at least one first rolling component includes a block rolling element and a seat rolling element.

The block rolling element is provided on a block rolling column provided on the inner surface of the block guard plate and rotates relative to the block rolling column.

The seat rolling element is provided on a seat rolling column provided on the inner surface of the seat guard plate and rotates relative to the seat rolling column.

An abdication space is formed between the block rolling element and the seat rolling element, and the abdication space is used to accommodate the driving element to pass through or stay in the case of deflection.

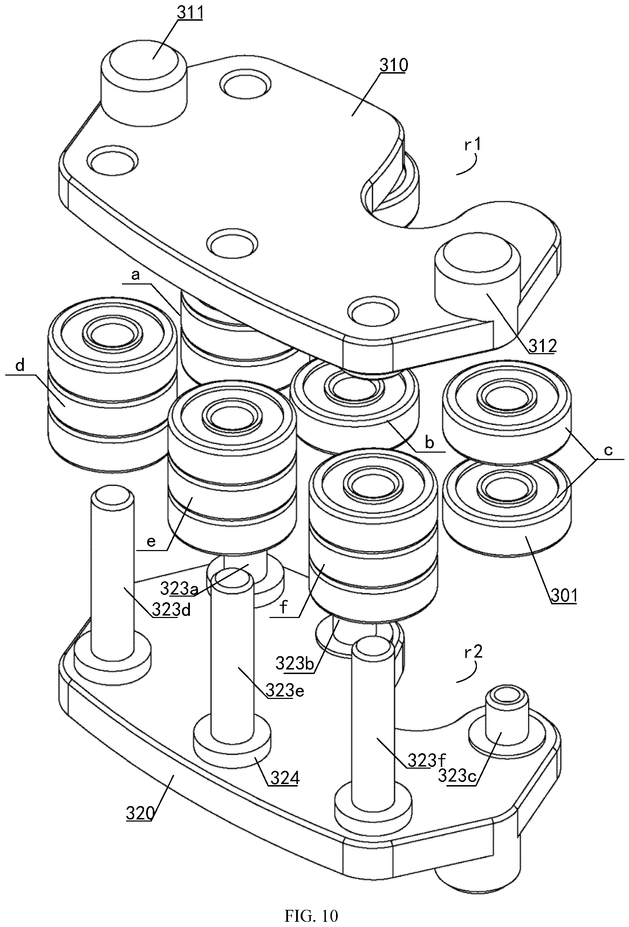

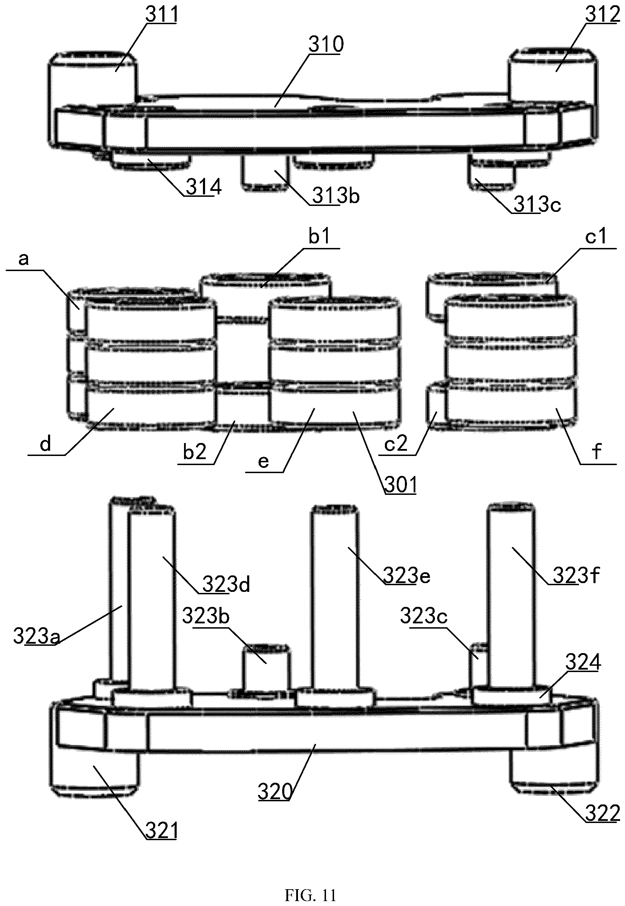

As shown in FIGS. 10 and 11, the first rolling component b may include a block rolling element b1 and a seat rolling element b2. The first rolling component c may include a block rolling element c1 and a seat rolling element c2. The block rolling element b1 is provided on a block rolling column 313b protruding on the inner surface of the block guard plate 310. The block rolling element c1 is provided on a block rolling column 313c protruding on the inner surface of the block guard plate 310. The seat rolling element b2 is provided on a seat rolling column 323b protruding on the inner surface of the seat guard plate 320. The seat rolling element c2 is provided on a seat rolling column 323c protruding on the inner surface of the seat guard plate 320.

For the rolling components 301, each rolling component 301 may be composed of one or more rolling elements. Each rolling element serves as a constituent unit of the rolling component 301, which may be a cylindrical structure, specifically a bearing structure, and each rolling element may have a middle through hole. The rolling element may be a block rolling element in contact with the inner surface of the block guard plate 310, or a seat rolling element in contact with the inner surface of the seat guard plate 320, specifically related to a contact or connection relationship between the rolling element and the block guard plate 310 or the seat guard plate 320. In addition, the rolling column is a cylinder that matches with the rolling element of the rolling component 301, used to penetrate the middle through hole of the rolling element, position a distribution of the rolling elements in the joint portion 103, and enable the rolling element to spin relative to the rolling column. The rolling column may be block rolling columns 313b and 313c that protrude from the inner surface of the block guard plate 310, or seat rolling columns 323b and 323c that protrude from the inner surface of the seat guard plate 320, specifically related to a contact or connection relationship between the rolling column and the block guard plate 310 or the seat guard plate 320.

A gap is formed between the block rolling element b1 and the seat rolling element b2, and a gap is also formed between the block rolling element c1 and the seat rolling element c2. These two gaps correspondingly form an abdication space, which is used to provide extra space for structural interference between the driving element 104 and the joint portion 103, so as to prevent interference effects between the driving element 104 and the joint portion 103 during deflection in the highly integrated structure or the initial state, thereby avoiding a limitation of the deflection control function of the deflection joint structure, ensuring that the stable deflection control may be achieved in the miniaturized and integrated deflection joint structure 100. As shown in FIG. 5, when the driving element 104 is in the initial state, the driving element 104 may interfere with the rolling components b and c in the gap between the block rolling element b1 and the seat rolling element b2 as well as the gap between the block rolling element c1 and the seat rolling element c2, corresponding to the rolling components b and c respectively. As shown in FIG. 7A, when the driving element 104 is in a retracted state, the driving element 104 may interfere with the rolling component c in the gap between the block rolling element c1 and the seat rolling element c2 corresponding to the rolling component c. Similarly, as shown in FIG. 8A, when the driving element 104 is in an extended state, the driving element 104 may interfere with the rolling component c in the gap between the block rolling element c1 and the seat rolling element c2 corresponding to the rolling component c. A width of the gap (i.e. a distance between the block rolling element and the corresponding seat rolling element) may match with a thickness of the driving element 104.

As shown in FIG. 5 to FIG. 11, according to an embodiment of the present disclosure, each of the at least one second rolling component includes at least one middle rolling element provided on at least one corresponding middle rolling column provided on the seat guard plate and rotating relative to the at least one middle rolling column.

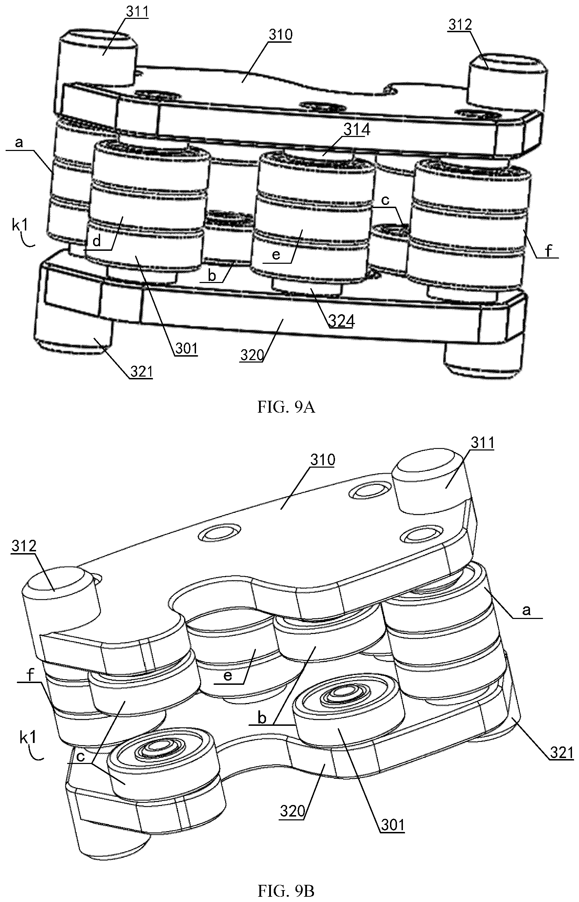

As shown in FIG. 9A to FIG. 11, any of the second rolling components (such as rolling components a, d, e, f) may include one or more middle rolling elements, each of which serves as a constituent unit of the second rolling component and may be a cylindrical structure, specifically a bearing structure, and each middle rolling element may have a middle through hole. A plurality of middle rolling elements may have the same structure as the block rolling element or seat rolling element of the first rolling component mentioned above, only distinguished in text due to different positions and functions. As shown in FIGS. 9A to 11, the second rolling component may have three middle rolling elements, which are arranged in series on the corresponding middle rolling column through their respective middle through holes. The middle rolling column may be longer than the block rolling column mentioned above, and a specific length of the middle rolling column may be a distance between the block guard plate 310 and the seat guard plate 320.

With the help of the middle rolling column and the middle through hole of each middle rolling element, a distribution of the middle rolling elements in the joint portion 103 may be positioned, and at the same time, the middle rolling element may spin relative to the middle rolling column. As shown in FIGS. 10 to 11, a plurality of middle rolling elements of the second rolling component a are arranged in series on a middle rolling column 323a, a plurality of middle rolling elements of the second rolling component d are arranged in series on a middle rolling column 323d, a plurality of middle rolling elements of the second rolling component e are arranged in series on a middle rolling column 323e, and a plurality of middle rolling elements of the second rolling component f are arranged in series on a middle rolling column 323f.

In this way, on the basis of the movable connection structure of the joint portion 103 relative to the first connecting portion 101 and the second connecting portion 102, a highly integration and structural miniaturization between the driving element 104 and the joint portion 103 is ensured, further ensuring that the operating component C may stably generate rolling friction, rather than sliding friction, when in contact with each rolling component in the joint portion 103, thereby providing a more reliable bending space and contact structure for the flexible bending of the operating component C.

As shown in FIG. 5 to FIG. 11, according to an embodiment of the present disclosure, the plurality of rolling components are respectively arranged in an arc shape at two side edges of the joint portion, along an extension direction of the operating component in the contact space and on two sides of the contact space.

As shown in FIGS. 5A, 7A and 8A, the rolling components a, b, and c are provided on a side of the contact space k2 along the extension direction of the operating component C; and the rolling components d, e, and f are provided on the other side of the contact space k2 along the extension direction of the operating component C. The rolling components a, b, and c are provided along an arc shape, that is, centers of the rolling components a, b, and c may be located within a same arc (such as three points sharing a circle). Similarly, the rolling components d, e, and f are also provided along an arc shape, that is, centers of the rolling components d, e, and f may also be located within a same arc (such as three points sharing a circle). The rolling components a, b, and c are provided along the edge of the joint portion 103 on a side of the contact space k2, while the rolling components d, e, and f are provided along the edge of the joint portion 103 on the other side of the contact space k2. Moreover, in the initial state shown in FIG. 5A, positions of the rolling components distributed in the middle among the plurality of rolling components are further away from the operating component C relative to the positions of the rolling components at two ends. In other words, the two side edges of the joint portion 103 are designed in a circular arc shape with two ends inward and the middle outward. In this way, the contact space k2 also presents a structural design with narrow ends and wide middle along the extension direction of the operating component C.

Therefore, when a deflection action occurs, inner edges of the plurality of rolling components d, e, and f on a side of the operating component C toward the contact space k2 may all be in tangential contact with the operating component C (as shown in FIG. 7A), or the inner edges of the plurality of rolling components a, b, and c on the other side of the operating component C toward the contact space k2 may all be in tangential contact with the operating component C (as shown in FIG. 8A), so that the plurality of rolling components on at least one side may simultaneously provide structural support for the operating component C with rolling friction effect when the operating component C undergoes flexible bending, effectively preventing the operating component from fails such as instability buckling.

It should be noted that the number of rolling components arranged in an arc shape on a side edge of the joint portion mentioned above is not limited to 3, but may also be 4 or more. In other words, an arrangement of rolling components is not limited to 3 points sharing a circle, but may also be a plurality of points sharing a circle, which is not specifically limited here.

As shown in FIG. 5 to FIG. 11, according to an embodiment of the present disclosure, the plurality of rolling components include two third rolling components and two fourth rolling components.

The two third rolling components are provided at an end of the joint portion close to the first connecting portion and arranged on two sides of an end of the contact space.

The two fourth rolling components are provided at the other end of the joint portion close to the second connecting portion and arranged on two sides of the other end of the contact space.

As shown in FIGS. 5A, 7A and 8A, in order to balance the number and position of rolling components so as to ensure a better contact between the operating component C and the rolling components with fewer rolling components in a limited space, it needs to be ensured that two third rolling components a and d are provided at the end of the joint portion 103 close to the deflection component A so as to limit the operating component C which enters the deflection component A. Similarly, it needs to be ensured that two fourth rolling components c and f are provided at the other end of the joint portion 103 close to the fixed component B so as to limit the operating component C which enters from the fixed component B. the two third rolling components a and d are respectively provided on two sides of the operating component C, and the two fourth rolling components c and f are also provided on two sides of the operating component C.

With the help of two third rolling components a and d and two fourth rolling components c and f, bending limit at two ends may be performed on the operating component C located in the contact space k2 of the joint portion 103, so as to prevent abnormal bending of the operating component C when bending occurs.

As shown in FIG. 5 to FIG. 11, according to an embodiment of the present disclosure, the plurality of rolling components further include two fifth rolling components. The two fifth rolling components are provided in the middle of the joint portion and arranged on two sides of the middle of the contact space.

A first spacing is formed between one of the two third rolling components and one of the two fifth rolling components close to the rotating secondary structure. A second spacing is formed between the other of the two fifth rolling components and the other of the two third rolling components, and the first spacing is less than the second spacing.

As shown in FIGS. 5A, 7A and 8A, in order to ensure stable rolling friction between the bending part of the operating component C and the rolling component 301 when the operating component C undergoes flexible bending, fifth rolling components b and e are respectively provided on two sides of the operating component C. One of the fifth rolling components b is located between the third rolling component a and the fourth rolling component c on a same side of the operating component C, and the other of the fifth rolling components e is located between the third rolling component d and the fourth rolling component f on a same side of the operating component C. That is, the fifth rolling components b and e are located in the middle position of the contact space k2 along the extension direction of the operating component C.

A first spacing is formed between the third rolling component a and the fifth rolling component b close to the rotating secondary structure mentioned above, and a second spacing is formed between the fifth rolling component e and the third rolling component d, and the first spacing is less than the second spacing. A spacing between the fifth rolling component e and the fourth rolling component f is equivalent to the second spacing mentioned above, and the spacing between the fifth rolling component b and the fourth rolling component c is generally greater than a third spacing so as to provide a setting space for the aforementioned abdication notch.

Therefore, with the help of the distribution settings of the third rolling components (a, d), fourth rolling components (c, f), and fifth rolling components (b, e) in the joint portion 103 mentioned above, the operating component C may generate stable rolling contact with each rolling component 301 of the joint portion 103 in the contact space, ensuring that the contact friction belongs to rolling friction, thereby ensuring that the deflection joint structure of the embodiment of the present disclosure may achieve the beneficial effect mentioned above.

It should be further noted that, as shown in FIGS. 9A, 10 and 11, in the embodiment of the present disclosure, an interval protrusion may be generally provided between each rolling component 301 and the inner surface of the corresponding block guard plate 310 or the seat guard plate 320, such as a ring columnar protrusion 314 provided on the inner surface of the block guard plate 310 and a ring columnar protrusion 324 provided on the inner surface of the seat guard plate 320. With such interval protrusion, the rolling component 301 is not easily in direct contact with the inner surface of the block guard plate 310 or the inner surface of the seat guard plate 320, which is conducive to a more stable and free rotation of the rolling component 301, avoiding the sliding friction. That is, the rolling component 301 is separated from the corresponding surface to avoid affecting the rotation of the rolling component 301.

Another aspect of the present disclosure provides a stapler, including the above-mentioned deflection joint structure, deflection component, fixed component, and operating component.

The deflection component is connected to the first connecting portion of the deflection joint structure.

The fixed component is connected to the second connecting portion of the deflection joint structure.

The operating component passes the contact space formed between the plurality of rolling components of the deflection joint structure.

In the case of deflection of the deflection component relative to the fixed component, at least one of the plurality of rolling components generates rolling friction with the operating component in the contact space.

The deflection component may be the jaw component of the stapler in the embodiment of the present disclosure. The fixed component may be a part of a handheld structure fixed relative to the jaw component. The operating component may be the cutting blade of the stapler, or it may specifically refer to the strip flexible and bendable handle of the cutting blade. The rolling components may be bearings.

Therefore, referring to the deflection joint structure 100 shown in FIGS. 1 to 11 in the above-mentioned embodiments of the present disclosure, it may be seen that compared to the jaw component of the stapler in traditional technologies in which sliding friction is generated between the protective wall and the handle of the cutting blade when the jaw component of the stapler performs stapling and cutting operations in case of deflection, the rolling friction between the handle of the cutting blade and the rolling components of the joint portion may be achieved in the embodiment of the present disclosure performs, thereby greatly reducing the contact frictional force of the handle of the cutting blade during the deflection process, without providing a greater axial force to the cutting blade at the handle. Even if the aspect ratio of the handle is greater, the reliable structural support for the handle may still be provided, significantly reducing the axial force the handle bears, preventing instability failure and improving the safety. Furthermore, the load-bearing capacity of each component may be reduced, which is beneficial for reducing the weight of the stapler, eliminating the need for the high-power motor, and reducing production costs and energy consumption. The deflection joint structure 100 of the stapler has a reliable structure, high safety, flexible and controllable rotation, high integration, and may achieve deflection with a small turning radius, which has extremely high commercial values.

Yet another aspect of the present disclosure provides a medical device including the above-mentioned deflection joint structure.

The medical device may be a device that requires any rotation or deflection functions, such as a medical robot, a medical prosthetic device, a medical joint device, a medical inspection device, or even a treatment device, which is not specifically limited by the applicant.

So far, a detailed description of the embodiments of the present disclosure has been provided in conjunction with the accompanying drawings.

The specific embodiments described above further describe the purposes, technical solutions, and beneficial effects of the present disclosure in detail. It should be understood that the above are only specific embodiments of the present disclosure and are not intended to limit the present disclosure. Any modifications, equivalent substitutions, improvements, etc. made within the spirit and principle of the present disclosure should be included in the scope of protection of the present disclosure.

## Claims

1. A deflection joint structure for achieving a deflection of a deflection component relative to a fixed component, comprising:
a first connecting portion provided on the deflection component;
a second connecting portion provided on the fixed component;
a joint portion, a part of the joint portion being provided in the first connecting portion, and another part of the joint portion being provided in the second connecting portion, wherein the joint portion comprises:
a plurality of rolling components arranged along a middle space in the joint portion so as to form a contact space for providing an operating component, wherein at least in a case of the deflection, at least one of the plurality of rolling components generates a rolling friction with the operating component in the contact space.

2. The deflection joint structure according to claim 1, wherein the first connecting portion comprises:
a deflection block provided above a part region of the joint portion;
a deflection seat provided below a part region of the joint portion; and
wherein a first setting space is formed between the deflection block and the deflection seat.

3. The deflection joint structure according to claim 2, wherein the deflection block comprises:
a block deflection hole penetrating an end portion of the deflection block away from the deflection component;
a block joint hole provided at the end portion close to the deflection component;
a block connecting column provided on a side of the end portion opposite to the block deflection hole.

4. The deflection joint structure according to claim 2, wherein the deflection seat comprises:
a seat deflection hole penetrating an end portion of the deflection seat away from the deflection component;
a seat joint hole provided at the end portion close to the deflection component;
a seat connecting hole provided on a side of the end portion opposite to the seat deflection hole.

5. The deflection joint structure according to claim 3, wherein the second connecting portion comprises:
a block deflection element corresponding to the deflection block and vertically provided on an end surface of an end portion of the fixed component close to the deflection component;
a seat deflection element corresponding to the deflection seat and provided symmetrically with the block deflection element on the end surface relative to the end surface; and
wherein a second setting space is formed between the block deflection element and the seat deflection element.

6. The deflection joint structure according to claim 5, wherein the block deflection element comprises:
a block deflection column provided in the block deflection hole of the deflection block, so that the block deflection element is located on an outer side of the deflection block.

7. The deflection joint structure according to claim 5, wherein the seat deflection element comprises:
a seat deflection column provided in a seat deflection hole of the deflection seat, so that the seat deflection element is located on an outer side of the deflection seat.

8. The deflection joint structure according to claim 1, wherein the joint portion comprises:
a block guard plate provided on a side of the plurality of rolling components;
a seat guard plate provided on the other side of the plurality of rolling components and forming the middle space of the joint portion.

9. The deflection joint structure according to claim 8, wherein the block guard plate comprises:
a first joint column corresponding to a block joint hole of a deflection block of the first connecting portion, wherein the first joint column is provided at an end portion of the block guard plate close to the first connecting portion;
a second joint column corresponding to a first defining groove of a block deflection element of the second connecting portion, wherein the second joint column is provided at the other end portion of the block guard plate close to the second connecting portion.

10. The deflection joint structure according to claim 8, wherein the seat guard plate comprises:
a third joint column corresponding to a seat joint hole of a deflection seat of the first connecting portion, wherein the third joint is provided at an end portion of the seat guard plate close to the first connecting portion;
a fourth joint column corresponding to a second defining groove of a seat deflection element of the second connecting portion, wherein the fourth joint is provided at the other end portion of the seat guard plate close to the second connecting portion.

11. The deflection joint structure according to claim 8, further comprising:
a driving element perpendicular to an end surface of an end portion of the fixed component close to the second connecting portion and movably connected to the first connecting portion, so as to drive the deflection component to deflect relative to the fixed component when extending or retracting relative to the second connecting portion.

12. The deflection joint structure according to claim 11, wherein the driving element further comprises:
a driving connection hole forming a penetrating relationship with a block connecting column of a deflection block of the first connecting portion, so that the block connecting column penetrates the driving connection hole, and further into a seat connecting hole of the deflection seat of the first connecting portion after, so as to form a rotating secondary structure between the first connecting portion and the second connecting portion.

13. The deflection joint structure according to claim 12, wherein the plurality of rolling components comprise:
at least one first rolling component corresponding to the rotating secondary structure, provided between the block guard plate and the seat guard plate, and located at an edge of the joint portion;
at least three second rolling components cooperated with the at least one first rolling component, provided between the block guard plate and the seat guard plate, and located at the edge of the joint portion; and
wherein the at least one first rolling component and the at least three second rolling components form the contact space located in the middle of the joint portion, and the contact space is configured to provide a space for the operating component to pass through and generate rolling friction with the plurality of rolling components in the case of the deflection.

14. The deflection joint structure according to claim 13, wherein the block guard plate comprises:
a first notch provided on the block guard plate and matching with the rotating secondary structure in the case of the deflection.

15. The deflection joint structure according to claim 14, wherein the seat guard plate comprises:
a second notch corresponding to the first notch, provided on the seat guard plate, and matching with the rotating secondary structure in the case of the deflection.

16. The deflection joint structure according to claim 13, wherein each of the at least one first rolling component comprises:
a block rolling element provided on a block rolling column provided on an inner surface of the block guard plate and rotating relative to the block rolling column;
a seat rolling element provided on a seat rolling column provided on an inner surface of the seat guard plate and rotating relative to the seat rolling column; and
wherein an abdication space is formed between the block rolling element and the seat rolling element, and the abdication space is configured to accommodate the driving element to pass through or stay in the case of the deflection.

17. The deflection joint structure according to claim 13, wherein each of the at least one second rolling component comprises:
at least one middle rolling element provided on at least one corresponding middle rolling column provided on the seat guard plate and rotating relative to the at least one middle rolling column.

18. The deflection joint structure according to claim 1, wherein the plurality of rolling components are respectively arranged in an arc shape at two side edges of the joint portion, along an extension direction of the operating component in the contact space and on two sides of the contact space.

19. A stapler, comprising:
the deflection joint structure of any one of claims 1 to 18;
the deflection component connected to the first connecting portion of the deflection joint structure;
the fixed component connected to the second connecting portion of the deflection joint structure;
the operating component passing the contact space formed between the plurality of rolling components of the deflection joint structure;
wherein in the case of the deflection of the deflection component relative to the fixed component, at least one of the plurality of rolling components generates rolling friction with the operating component in the contact space.

20. A medical device, comprising the deflection joint structure of any one of claims 1 to 18.
